# FASCICULE DE BREVET EUROPEEN

(11) **EP 3 974 515 B1**
(45) Date de publication et mention de la délivrance du brevet: **08.03.2023**
(21) Numéro de dépôt: 21198293.9
(22) Date de dépôt: 22.09.2021
(51) Int. Cl.: C12M 1/107, C12M 1/00

(54) **CONDUIT DOUBLE VANNES POUR L ÉVACUATION DE LA PARTIE LIQUIDE PRÉSENTE DANS UN CIRCUIT DE CIRCULATION DE BIOGAZ**
DOPPELVENTILROHR FÜR DIE ABLEITUNG DES FLÜSSIGEN ANTEILS IN EINEM BIOGASKREISLAUF
DOUBLE-VALVE CONDUIT FOR DISCHARGE OF THE LIQUID PORTION PRESENT IN A BIOGAS CIRCULATION CIRCUIT

(30) Priorité: 28.09.2020 FR 2009828
(43) Date de publication de la demande: 30.03.2022
(73) Titulaire: ADG - Ateliers Des Graves, 31270 Villeneuve Tolosane (FR)
(72) Inventeur: OF, Julien, 31270 VILLENEUVE TOLOSANE (FR); MORISAN, Richard, 31270 VILLENEUVE TOLOSANE (FR)
(74) Mandataire: Argyma

(56) Documents cités:
- EP-A1- 1 820 782
- WO-A2-2011/055972
- FR-A1- 3 043 397
- GB-A- 2 251 054

## Description

### Domaine technique

La présente invention se rapporte au domaine de la méthanisation et concerne plus particulièrement un système de méthanisation et un procédé d'évacuation de liquide. Le système de méthanisation selon l'invention permet notamment d'évacuer les liquides circulant dans le conduit d'acheminement du biogaz en sortie de méthaniseur.

### Etat de la technique

La méthanisation des matières organiques est un processus naturel connu qui permet de transformer des matières organiques, notamment des matières agricoles, en biogaz. La méthanisation s'effectue par dégradation biologique des matières organiques par un consortium microbien comportant des bactéries. Il est ainsi connu de transformer en biogaz la fraction fermentescible des effluents d'élevage en disposant ces effluents dans un méthaniseur.

Un type connu de méthaniseur comprend une cuve ouverte (appelée digesteur), dans laquelle on place les effluents, et un dispositif de collecte de biogaz, disposé dans ladite cuve. Le méthaniseur comporte une membrane souple, appelée couverture, qui s'étend au-dessus des matières organiques stockées dans la cuve afin de collecter le biogaz produit par la fraction fermentescible des effluents. Un conduit d'acheminement est relié fluidiquement à la couverture afin d'acheminer le biogaz hors du méthaniseur pour l'exploiter. Ce faisant, on constate qu'un mélange de condensats issu de la méthanisation, comportant notamment de l'eau, se retrouve en phase liquide et gazeuse dans le conduit d'acheminement, ce qui augmente le taux d'humidité du flux de biogaz, qui peut alors atteindre 100 % dans certains cas.

Or, certains équipements de traitement du biogaz tels que, par exemple, les modules de cogénération, nécessitent de limiter le taux d'humidité du flux de biogaz en-dessous d'un seuil prédéterminé, par exemple de l'ordre de 33%.

Une première solution connue pour remédier à ce problème consiste à installer un module de séchage ou « dessécheur ». Cependant, un tel module peut s'avérer particulièrement onéreux et complexifie le système et sa maintenance.

Une deuxième solution connue consiste à utiliser un pot à condensats permettant de collecter les condensats en phase liquide. Ce type de dispositif fonctionne sur le principe du trop-plein et nécessite d'être monté dans le conduit d'acheminement, ce qui augmente le coût et la complexité du système ainsi que sa maintenance. En outre, ce type de dispositif nécessite un espace important, ce qui présente un autre inconvénient. FR 3 043 397 divulgue un méthanisateur avec une système de collecte et de recirculation du biogas.

Il existe donc le besoin d'une solution permettant de remédier au moins en partie à ces inconvénients.

### Exposé de l'invention

L'invention vise à résoudre au moins en partie ces inconvénients en proposant une solution de module de couverture qui soit à la fois simple, fiable, efficace, peu onéreuse, aisée et rapide à monter, notamment sur un système de méthanisation existant.

A cet effet, l'invention a tout d'abord pour objet un système de méthanisation de matières organiques, ledit système comprenant un méthaniseur, un conduit d'acheminement, un conduit d'évacuation, une première vanne montée dans ledit conduit d'évacuation et un détecteur, ledit conduit d'acheminement étant relié fluidiquement au méthaniseur afin d'évacuer le biogaz produit par ledit méthaniseur, ledit conduit d'évacuation étant relié fluidiquement au conduit d'acheminement afin de collecter la partie liquide circulant dans le conduit d'acheminement et de la stocker, notamment au-dessus de la première vanne lorsque ladite première vanne est fermée ou au-dessus de la deuxième vanne lorsque la deuxième vanne est fermée et la première vanne est ouverte, ledit détecteur comprenant un flotteur, placé à l'intérieur du conduit d'évacuation et qui est apte à flotter à la surface de la partie liquide collectée dans ledit conduit d'évacuation, et un élément sensible, monté en amont de la première vanne et qui est apte à détecter le flotteur lorsque ledit flotteur flotte au droit dudit élément sensible, au niveau d'un seuil de déclenchement de purge prédéterminé, afin de permettre l'évacuation d'au moins une fraction de la partie liquide stockée dans le conduit d'évacuation, ledit système de méthanisation étant remarquable en ce qu'il comprend une deuxième vanne montée en aval de la première vanne de sorte à former un sas de stockage de la fraction de partie liquide entre la première vanne et la deuxième vanne, ledit sas de stockage étant fluidiquement indépendant du conduit d'acheminement lorsque la première vanne est fermée.

Le sas de stockage permet donc de réaliser la purge du liquide en deux temps en remplissant tout d'abord le sas, en isolant fluidiquement le sas du conduit d'acheminement en fermant la première vanne, la deuxième vanne étant fermée, puis en évacuant le liquide en ouvrant la deuxième vanne dans un second temps. Ainsi, en gardant au moins une de la première vanne ou de la deuxième vanne fermée à tout instant, le sas permet notamment d'éviter au biogaz de s'échapper du conduit d'acheminement lors de la vidange du liquide, rendant ainsi l'installation plus sécurisée pour les opérateurs tout en préservant le rendement.

Une solution alternative aurait été de connecter un conduit d'évacuation vertical fermé par une seule vanne sous le conduit d'acheminement mais une fraction du biogaz circulant dans le conduit d'acheminement aurait pu s'échapper lorsque la vanne est ouverte pour évacuer le liquide, ce qui aurait constitué une perte de biogaz (baisse de rendement du système) et aurait pu s'avérer particulièrement dangereux du fait du potentiel inflammable du biogaz.

Le remplissage du sas peut se faire de deux façons : soit la première vanne reste ouverte et la deuxième vanne reste fermée le temps du remplissage jusqu'à ce que le niveau de liquide monte suffisamment pour que le flotteur soit détecté puis la première vanne est fermée et la deuxième vanne est ouverte pour vidanger, soit la première vanne reste fermée le temps du remplissage jusqu'à ce que le niveau de liquide monte suffisamment pour que le flotteur soit détecté puis, la deuxième vanne étant fermé, on ouvre la première vanne afin de remplir le sas, puis on la ferme et on ouvre la deuxième vanne pour vidanger.

Dans le deuxième cas de figure :
- dans une forme de réalisation, le volume du sas de stockage est supérieur au volume de la partie liquide stockée en amont de la première vanne entre ladite première vanne et le seuil de déclenchement de purge prédéterminé afin de permettre la purge de tout le volume de liquide stocké au-dessus de la première vanne ;
- dans une autre forme de réalisation, le volume du sas de stockage est égal au volume de la partie liquide stockée en amont de la première vanne entre ladite première vanne et le seuil de déclenchement de purge prédéterminé afin que la partie liquide stockée en amont de la première vanne remplisse entièrement et exactement le sas de stockage, évitant ainsi que du biogaz ne rentre dans le sas de stockage tout en permettant la purge de tout le volume de liquide stocké en amont de la première vanne ;
- dans une autre forme de réalisation, le volume du sas de stockage est inférieur au volume de la partie liquide stockée en amont de la première vanne entre ladite première vanne et le seuil de déclenchement de purge prédéterminé afin d'éviter la formation d'un espace libre dans le sas de stockage lors de son remplissage, évitant ainsi au biogaz de pénétrer dans le sas de stockage.

Dans une forme de réalisation, la première vanne et la deuxième vanne sont configurées pour être commandées à distance.

Dans une forme de réalisation, la première vanne et la deuxième vanne sont des électrovannes.

De manière préférée, le système comprend un module de contrôle apte à recevoir un signal de détection envoyé par le détecteur lorsque l'élément sensible détecte le flotteur et à commander l'ouverture et la fermeture de la première vanne et de la deuxième vanne, de préférence de manière automatisée, afin d'effectuer la purge.

Dans une forme de réalisation, le module de contrôle est configuré pour, à réception du signal de détection, fermer la première vanne pour isoler le sas de stockage du conduit d'acheminement, ouvrir la deuxième vanne lorsque la première vanne a été fermée afin d'évacuer au moins une fraction de la partie liquide stockée dans le sas de stockage, fermer la deuxième vanne lorsque l'au moins une fraction de partie liquide a été évacuée du sas de stockage et ouvrir la première vanne afin de stocker une nouvelle fraction de partie liquide dans le sas de stockage.

Dans une autre forme de réalisation, le module de contrôle est configuré pour, à réception du signal de détection, la deuxième vanne étant fermée, ouvrir la première vanne afin de remplir le sas de stockage avec au moins une fraction de la partie liquide, fermer la première vanne, ouvrir la deuxième vanne lorsque la première vanne a été fermée afin d'évacuer l'au moins une fraction de partie liquide stockée dans le sas de stockage et fermer la deuxième vanne lorsque l'au moins une fraction de partie liquide a été évacuée du sas de stockage.

Dans une forme de réalisation, le système comprend un module de traitement relié au conduit d'acheminement afin de collecter le biogaz produit par le méthaniseur. Ce module de traitement peut par exemple être un filtre à charbon actif, un module de cogénération, un réservoir de stockage ou tout autre module adapté.

De préférence, le conduit d'évacuation est relié à la partie inférieure du conduit d'acheminement afin de collecter la partie liquide par gravité. Par exemple, le conduit d'évacuation peut comprendre une portion de liaison au conduit d'acheminement qui s'étend sensiblement verticalement vers le bas sous conduit d'acheminement afin d'évacuer la partie liquide facilement.

De manière avantageuse, le conduit d'acheminement comprend un point bas, par exemple en étant coudé, et le conduit d'évacuation est connecté fluidiquement audit conduit d'acheminement au niveau dudit point bas afin d'acheminer la partie liquide du conduit d'acheminement vers le conduit d'évacuation par gravité.

L'invention concerne également un procédé d'évacuation de la partie liquide circulant dans le conduit d'acheminement d'un système de méthanisation tel que décrit ci-avant, ledit procédé comprenant, la première vanne étant ouverte et la deuxième vanne étant fermée, les étapes de :
- collecte de la partie liquide dans le sas de stockage,
- détection du flotteur par l'élément sensible du détecteur,
- fermeture de la première vanne,
- ouverture de la deuxième vanne afin d'évacuer la partie liquide stockée dans le sas de stockage,
- fermeture de la deuxième vanne,
- ouverture de la première vanne afin de permettre de nouveau le stockage de la partie liquide dans le sas de stockage.

Dans un mode de réalisation, le système comprenant un module de contrôle, le procédé comprend une étape d'envoi d'un signal de détection par le détecteur audit module de contrôle lorsque le flotteur a été détecté et, à réception dudit signal de détection, la commande par le module de contrôle de la fermeture de la première vanne puis de l'ouverture de la deuxième vanne puis de la fermeture de la deuxième vanne et enfin de la fermeture de la première vanne.

Avantageusement, le module de contrôle est configuré pour réaliser séquentiellement la fermeture de la première vanne, l'ouverture de la deuxième vanne, la fermeture de la deuxième vanne et l'ouverture de la première vanne de préférence de manière automatique.

L'invention concerne également un procédé d'évacuation de la partie liquide circulant dans le conduit d'acheminement d'un système de méthanisation tel que décrit ci-avant, ledit procédé comprenant, la première vanne et la deuxième vanne étant fermées, les étapes de :
- collecte de la partie liquide dans le conduit d'évacuation en amont de la première vanne,
- détection du flotteur par l'élément sensible du détecteur,
- ouverture de la première vanne, la deuxième vanne étant fermée,
- stockage de la partie liquide dans le sas de stockage,
- fermeture de la première vanne,
- une fois la première vanne fermée, ouverture de la deuxième vanne afin d'évacuer la partie liquide stockée dans le sas de stockage,
- fermeture de la deuxième vanne.

Dans un mode de réalisation, le système comprenant un module de contrôle, le procédé comprend une étape d'envoi d'un signal de détection par le détecteur audit module de contrôle lorsque le flotteur a été détecté et, à réception dudit signal de détection, la commande par le module de contrôle de l'ouverture de la première vanne puis de la fermeture de la première vanne puis de l'ouverture de la deuxième vanne.

Avantageusement, le module de contrôle est configuré pour réaliser séquentiellement l'ouverture de la première vanne, la fermeture de la première vanne, l'ouverture de la deuxième vanne et la fermeture de la deuxième vanne, de préférence de manière automatique.

### Description des figures

D'autres caractéristiques et avantages de l'invention apparaîtront encore à la lecture de la description qui va suivre. Celle-ci est purement illustrative et doit être lue en regard des dessins annexés sur lesquels :
[Fig. 1] La figure 1 illustre schématiquement une forme de réalisation du système selon l'invention.
[Fig. 2] La figure 2 est une vue schématique partielle d'une première forme de réalisation du conduit d'évacuation lors de la collecte de la partie liquide provenant du conduit d'acheminement.
[Fig. 3] La figure 3 est une vue schématique partielle du conduit d'évacuation de la figure 2 lorsque le flotteur atteint le seuil de déclenchement de purge prédéterminé et qu'il est alors détecté par l'élément sensible.
[Fig. 4] La figure 4 est une vue schématique partielle du conduit d'évacuation de la figure 3 une fois la première vanne fermée.
[Fig. 5] La figure 5 est une vue schématique partielle du conduit d'évacuation de la figure 4 une fois la deuxième vanne ouverte.
[Fig. 6] La figure 6 illustre schématiquement un premier mode de réalisation du procédé selon l'invention.
[Fig. 7] La figure 7 est une vue schématique partielle d'une deuxième forme de réalisation du conduit d'évacuation lors de la collecte de la partie liquide provenant du conduit d'acheminement.
[Fig. 8] La figure 8 est une vue schématique partielle du conduit d'évacuation de la figure 7 lorsque le flotteur atteint le seuil de déclenchement de purge prédéterminé et qu'il est alors détecté par l'élément sensible.
[Fig. 9] La figure 9 est une vue schématique partielle du conduit d'évacuation de la figure 8 une fois la première vanne ouverte.
[Fig. 10] La figure 10 est une vue schématique partielle du conduit d'évacuation de la figure 9 une fois la première vanne fermée, la deuxième vanne étant toujours fermée.
[Fig. 11] La figure 11 est une vue schématique partielle du conduit d'évacuation de la figure 10 une fois la deuxième vanne ouverte, la première vanne étant toujours fermée.
[Fig. 12] La figure 12 illustre schématiquement un deuxième mode de réalisation du procédé selon l'invention.

### Description détaillée d'au moins un mode de réalisation

Le système selon l'invention permet à la fois de capter le biogaz produit par la transformation de la fraction fermentescible de matières organiques et d'évacuer la partie liquide afin d'éviter qu'elle ne pénètre dans les équipements avals de traitement du biogaz.

Les matières organiques peuvent notamment être des effluents d'élevage comme du lisier de bovin, de palmipède, de porcin, ou de toute autre matière organique dont une fraction fermentescible est apte à être transformée en biogaz.

### Système 1

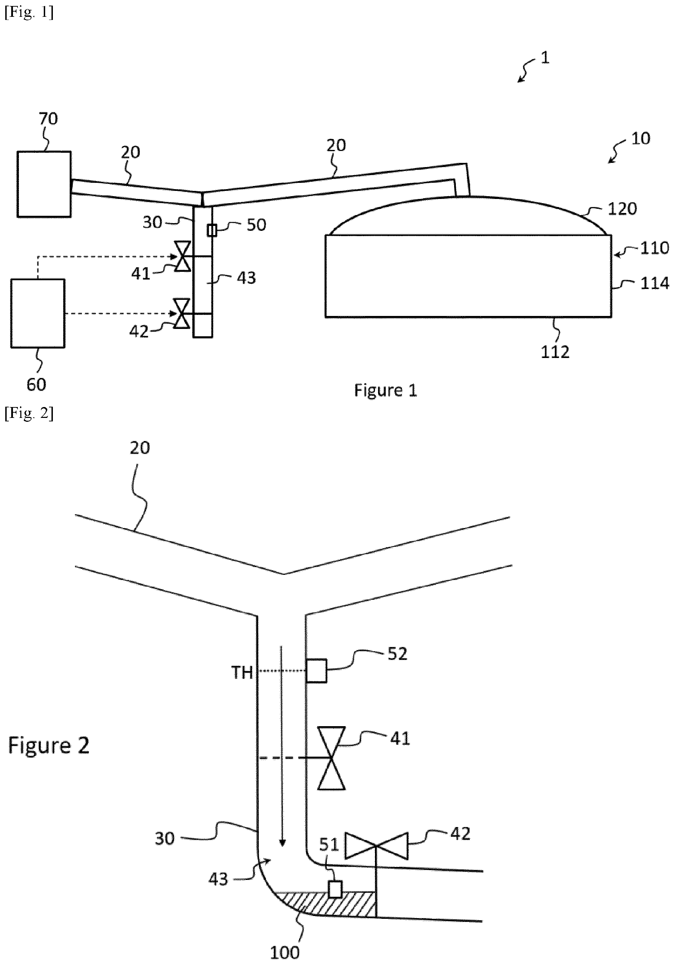

En référence à la figure 1, le système 1 selon l'invention comprend un méthaniseur 10, un conduit d'acheminement 20, un conduit d'évacuation 30, une première vanne 41, une deuxième vanne 42, un détecteur 50 et un module de contrôle 60. Dans cet exemple, le système 1 comprend en outre un module de traitement 70.

### Méthaniseur 10

Le méthaniseur 10 comprend une cuve 110 destinée à recevoir des matières organiques (non visibles sur la figure 1) et un module de couverture 120 de ladite cuve 110.

### Cuve 110

Dans cet exemple non limitatif, la cuve 110 comprend un fond 112 et au moins une paroi 114 latérale s'étendant verticalement dudit fond 112 et délimitant une ouverture au niveau de sa partie supérieure. La cuve 110 peut par exemple être enterrée dans le sol et être réalisée en béton ou en géotextile ou tout autre matériau adapté.

La cuve 110 présente dans cet exemple une forme cylindrique de section circulaire et comporte une unique paroi 114 latérale. Dans toute autre forme de réalisation, la cuve 110 pourrait présenter une autre forme, par exemple parallélépipédique en comportant quatre parois latérales.

### Module de couverture 120

Le module de couverture 120 est destiné à reposer sur les matières organiques contenues dans la cuve 110. Plus précisément, le module de couverture 120 est configuré pour recouvrir l'ouverture supérieure de la cuve 110 de sorte à épouser la forme de la paroi 114 tout en autorisant la formation d'un espace interne permettant de collecter le biogaz afin de l'évacuer par le conduit d'acheminement 20. On notera que la forme du module de couverture 120 est définie par la forme de la cuve 110. Elle est ainsi de forme circulaire dans l'exemple de la figure 1.

Le module de couverture 120 de la cuve 110 comprend par exemple une membrane souple s'étendant au-dessus des matières organiques dans l'ouverture supérieure délimitée par la paroi 114 de la cuve 110. La membrane 122 souple est apte à former un dôme au-dessus des matières organiques afin de concentrer le biogaz produit par la fermentation desdites matières organiques.

### Conduit d'acheminement 20

L'entrée du conduit d'acheminement 20 est relié fluidiquement au module de couverture 120 afin d'évacuer le biogaz produit dans la cuve 110 et stocké sous le module de couverture 120. La sortie du conduit d'acheminement 20 est connectée au module de traitement 70.

Dans cet exemple, le conduit d'acheminement 20 est fixé à la partie centrale de la membrane, celle-ci étant perforée en son centre pour autoriser le biogaz à circuler du module de couverture 120 jusqu'au module de traitement 70 via le conduit d'acheminement 20.

Afin de permettre l'évacuation de la partie liquide circulant dans le conduit d'acheminement 20, notamment issue de la condensation des gaz de méthanisation, le conduit d'acheminement 20 est avantageusement conformé de manière à présenter un point bas au niveau duquel est fixé le conduit d'évacuation 30 afin de conduire la partie liquide vers le conduit d'évacuation 30 par gravité. Par exemple, le conduit d'acheminement peut présenter une forme de V, comme illustré sur les figures 2 à 5 ou 7 à 10, ou de U ou toute autre forme adaptée permettant de conduire la partie liquide vers le conduit d'évacuation 30.

### Conduit d'évacuation 30

Le conduit d'évacuation 30 comprend une portion sensiblement verticale reliée fluidiquement au point bas du conduit d'acheminement 20 afin de collecter la partie liquide circulant dans ledit conduit d'acheminement 20.

Dans une première forme de réalisation illustrée sur les figures 2 à 5, le conduit d'évacuation 30 présente une courbure supérieure à 90° et une portion de sortie inclinée de quelques degrés, par exemple de 3°, par rapport à l'horizontale terrestre afin de permettre une évacuation vers l'extérieur du système 1 selon une pente douce par gravité.

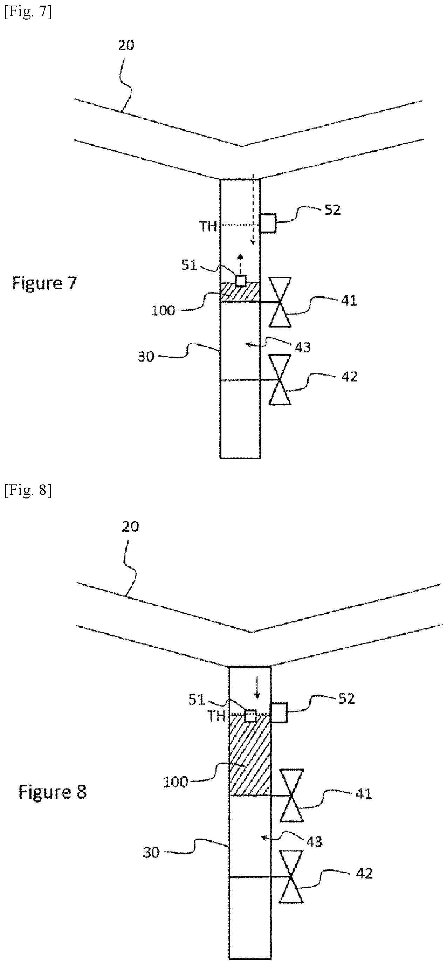

Dans une deuxième forme de réalisation illustrée sur les figures 7 à 10, le conduit d'évacuation 30 est droit et vertical afin de permettre une évacuation rapide par gravité.

Dans une autre forme de réalisation, le conduit d'évacuation 30 pourrait présenter toute autre forme adaptée.

### Première vanne 41

La première vanne 41 est montée dans le conduit d'évacuation 30 et est configurée pour être placée dans une position fermée dans laquelle ladite première vanne 41 empêche la partie liquide de circuler dans le conduit d'évacuation 30 au-delà de ladite première vanne 41 et une position ouverte dans laquelle ladite première vanne 41 permet à la partie liquide de circuler dans le conduit d'évacuation 30 en direction de la deuxième vanne 42.

De préférence, la première vanne 41 est une électrovanne.

Dans cet exemple, la première vanne 41 est apte à être commandée à distance en ouverture ou en fermeture par le module de contrôle 60, par exemple de manière filaire ou sans fil.

Deuxième vanne 42

La deuxième vanne 42 est montée dans le conduit d'évacuation 30 en aval de la première vanne 41 de sorte à former un sas de stockage 43 de la partie liquide entre la première vanne 41 et la deuxième vanne 42, ledit sas de stockage 43 étant fluidiquement indépendant du conduit d'acheminement 20 lorsque la première vanne 41 est en position fermée.

La deuxième vanne 42 est configurée pour être placée dans une position fermée dans laquelle ladite deuxième vanne 42 empêche la partie liquide située dans le sas de stockage 43 de circuler vers l'aval du conduit d'évacuation 30 et une position ouverte dans laquelle ladite deuxième vanne 42 permet à la partie liquide située dans le sas de stockage 43 de circuler dans le conduit d'évacuation 30 à travers la deuxième vanne 42 pour être évacuée, par exemple vers un réseau d'évacuation d'eaux usées.

De préférence, la deuxième vanne 42 est une électrovanne.

Dans cet exemple, la deuxième vanne 42 est apte à être commandée à distance en ouverture ou en fermeture par le module de contrôle 60, par exemple de manière filaire ou sans fil.

### Détecteur 50

Le détecteur 50 est configuré pour détecter que le niveau de liquide 100 collecté dans le conduit d'évacuation 30 a atteint un seuil de déclenchement de purge TH prédéterminé et pour alerter le module de contrôle 60 afin de réaliser la purge du sas de stockage 43.

Dans cet exemple, le détecteur 50 est un capteur magnétique comprenant un flotteur 51 et un élément sensible 52.

Le flotteur 51 est placé dans le conduit d'évacuation 30 et est apte à flotter à la surface de la partie liquide 100 stockée dans le conduit d'évacuation 30 au-dessus de la première vanne 41 ou au-dessus de la deuxième vanne 42.

Dans cet exemple préféré, l'élément sensible 52 est monté au-dessus de la première vanne 41, au niveau du seuil de déclenchement de purge TH prédéterminé, et est apte à détecter le flotteur 51 lorsque ledit flotteur 51 flotte au droit dudit élément sensible 52 et à envoyer un signal de détection au module de contrôle 60 lorsque le flotteur 51 a été détecté au niveau du seuil de déclenchement de purge TH prédéterminé. La position de l'élément sensible 52, notamment par rapport à la première vanne 41, peut être adaptée en fonction du moment auquel on souhaite réaliser la purge.

### Module de contrôle 60

Le module de contrôle 60 est configuré pour recevoir le signal de détection envoyé par le détecteur 50 et pour commander la première vanne 41 et la deuxième vanne 42 en ouverture et en fermeture.

Dans une forme de réalisation, le module de contrôle 60 est configuré pour :
- à réception du signal de détection, fermer la première vanne 41 afin d'isoler fluidiquement le sas de stockage 43 du conduit d'acheminement 20,
- ouvrir la deuxième vanne 42 une fois la première vanne 41 fermée afin d'évacuer l'au moins une fraction de partie liquide 100 stockée dans le sas de stockage 43,
- fermer la deuxième vanne 42 lorsque l'au moins une fraction de partie liquide 100 a été évacuée du sas de stockage 43, et
- ouvrir la première vanne 41 afin de stocker une nouvelle fraction de partie liquide 100 dans le sas de stockage 43.

Dans une autre forme de réalisation, le module de contrôle 60 est configuré pour :
- à réception du signal de détection, ouvrir la première vanne 41 pour remplir le sas de stockage 43 avec au moins une fraction de la partie liquide 100, la deuxième vanne 42 étant fermée,
- fermer la première vanne 41,
- ouvrir la deuxième vanne 42 une fois la première vanne 41 fermée afin d'évacuer l'au moins une fraction de partie liquide 100 stockée dans le sas de stockage 43, et
- fermer la deuxième vanne 42 lorsque l'au moins une fraction de partie liquide 100 a été évacuée du sas de stockage 43.

### Module de traitement 70

Le module de traitement 70 permet de traiter le biogaz produit par le méthaniseur 10. Par exemple, le module de traitement 70 peut être un filtre à charbon actif permettant de désulfurer le biogaz produit par le méthaniseur 10 afin par exemple d'en permettre la combustion dans un module de cogénération et produire ainsi de l'électricité et de la chaleur. Le module de traitement 70 peut aussi être un module de cogénération, un réservoir de stockage de biogaz ou tout autre module de traitement de biogaz connu.

### Mise en oeuvre de l'invention

L'opération de purge du système 1 va être décrite dans deux modes de réalisation.

Le premier mode est décrit en référence aux figures 2 à 6.

Dans ce premier mode, la première vanne 41 est ouverte et la deuxième vanne 42 est fermée à l'état initial de repos, c'est-à-dire hors des moments de purge, afin de collecter la partie liquide 100 provenant du conduit d'acheminement 20 directement dans le sas de stockage 43 dans une étape E1.

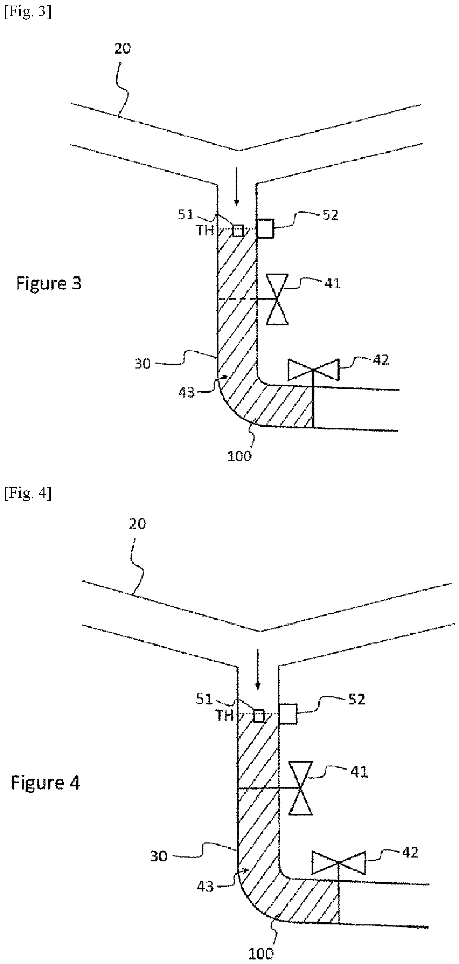

Lorsque le flotteur 51 se retrouve au même niveau que l'élément sensible 52, c'est-à-dire au niveau du seuil de déclenchement de purge TH prédéterminé, comme illustré sur la figure 3, l'élément sensible 52 détecte le flotteur 51 dans une étape E2, ce qui signifie que le niveau de la partie liquide 100 a atteint le seuil de déclenchement de purge TH.

Dans ce cas, le détecteur 50 envoie un signal de détection au module de contrôle 60 puis le module de contrôle 60 commande, de préférence automatiquement, la première vanne 41 et la deuxième vanne 42 à distance ou bien informe un opérateur, par exemple via un signal d'alerte, afin que l'opérateur aille manuellement manipuler la première vanne 41 et la deuxième vanne 42.

A réception du signal de détection, la première vanne 41 est tout d'abord commandée en fermeture dans une étape E3 (figure 4), la deuxième vanne 42 étant toujours fermée, de sorte à isoler le sas de stockage 43 du conduit d'acheminement 20. Ce faisant, une fraction de la partie liquide 100 correspondant au volume du sas de stockage 43 remplit entièrement ledit volume du sas de stockage 43 de sorte que le biogaz ne puisse circuler dans le sas de stockage 43 et s'échapper du conduit d'évacuation 30.

Lorsque la première vanne 41 est commandée par le module de contrôle 60, la première vanne 41 peut envoyer un message de confirmation de fermeture au module de contrôle 60 une fois ladite première vanne 41 fermée afin de permettre au module de contrôle 60 de poursuivre la suite de la purge.

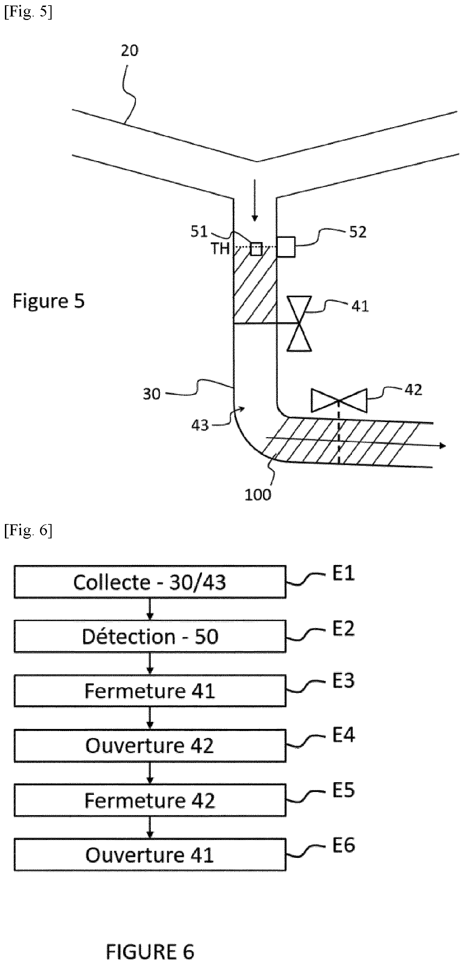

Ensuite, dans une étape E4, la deuxième vanne 41 est commandée en ouverture (figure 5) afin de vider le sas de stockage 43 de la fraction de partie liquide 100 qu'elle contient.

Une fois le sas de stockage 43 vidé, la deuxième vanne 42 est refermée dans une étape E5. Lorsque la deuxième vanne 42 est commandée par le module de contrôle 60, la deuxième vanne 42 peut envoyer un message de confirmation de fermeture au module de contrôle 60 une fois ladite deuxième vanne 42 refermée afin de permettre au module de contrôle 60 d'achever l'opération et remettre le système 1 dans son état initial, c'est-à-dire hors purge.

Une fois la deuxième vanne 42 refermée, la première vanne 41 est rouverte dans une étape E6 de sorte remettre le système 1 dans son état initial, c'est-à-dire hors purge, afin de pouvoir reprendre la collecte de liquide dans le sas de stockage 43 (étape E1, figure 2).

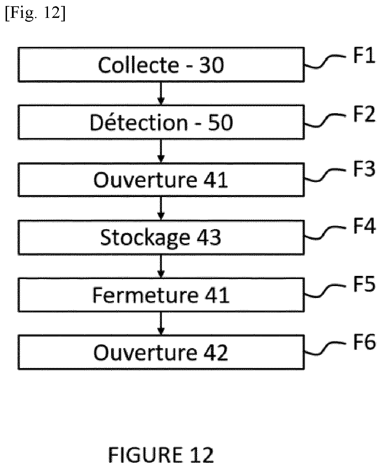

Le deuxième mode de réalisation est décrit en référence aux figures 8 à 12.

Dans ce deuxième mode, à l'état initial hors-purge (repos), la première vanne 41 est fermée et la deuxième vanne 42 est de préférence fermée, comme illustré sur la figure 7.

Au fur et à mesure que la partie liquide 100 circulant dans le conduit d'acheminement 20 remplit le conduit d'évacuation 30 au-dessus de la première vanne 41 dans une étape F1 (figure 7), le flotteur 51 se déplace avec la surface de la partie liquide 100 stockée dans le conduit d'évacuation 30 en amont de la première vanne 41 qui est fermée.

Lorsque le flotteur 51 se retrouve au même niveau que l'élément sensible 52, c'est-à-dire au niveau du seuil de déclenchement de purge TH prédéterminé, comme illustré sur la figure 8, l'élément sensible 52 détecte le flotteur 51 et en déduit que le niveau de la partie liquide a atteint le seuil de déclenchement de purge TH prédéterminé dans une étape F2. Dans ce cas, le détecteur 50 envoie un signal de détection au module de contrôle 60 puis le module de contrôle 60 commande, de préférence automatiquement, la première vanne 41 et la deuxième vanne 42 à distance ou bien informe un opérateur, par exemple via un signal d'alerte, afin que l'opérateur aille manuellement manipuler la première vanne 41 et la deuxième vanne 42.

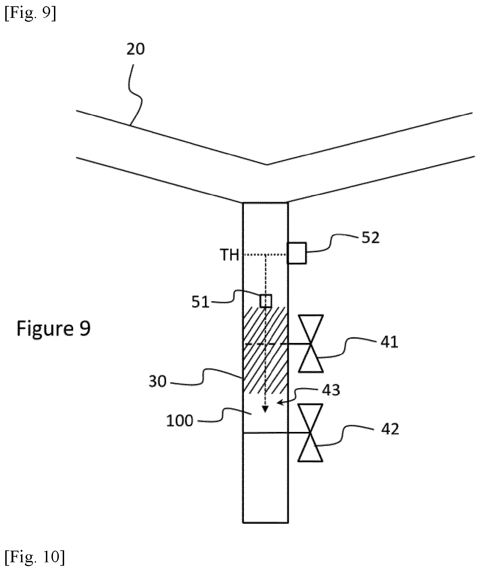

A réception du signal de détection, la première vanne 41 est ouverte dans une étape F3 ne sorte à permettre la circulation d'au moins une fraction de la partie liquide 100 vers le sas de stockage 43 dans une étape F4 (figure 9).

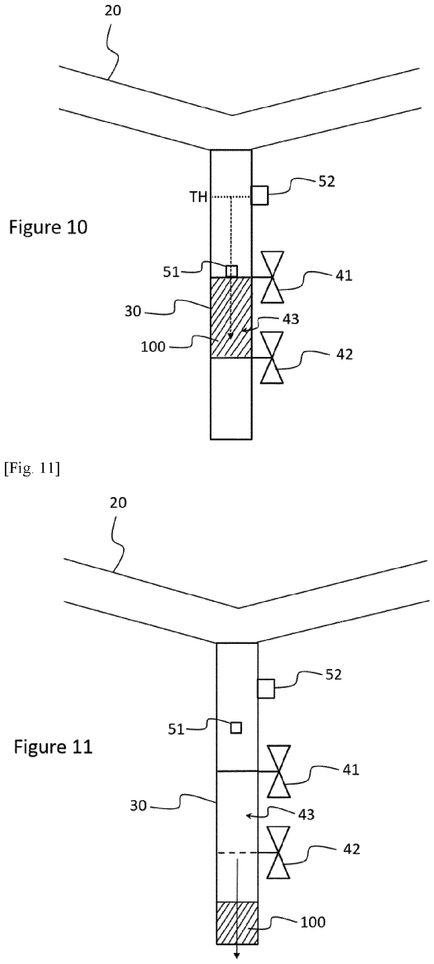

Lorsque le sas de stockage 43 est rempli ou bien que toute la partie liquide 100 s'est écoulée dans le sas de stockage 43, la première vanne 41 est fermée dans une étape F5 afin d'isoler fluidiquement le sas de stockage 43 du conduit d'acheminement 20 et éviter ainsi au biogaz de s'échapper du système 1 (figure 10).

Dans ce deuxième mode, il est préférable de configurer le seuil de déclenchement de purge TH de manière à ce que le volume stocké au-dessus de la première vanne 41 (étape F1) soit suffisant pour remplir entièrement le sas de stockage 43 et éviter ainsi le passage d'un petit volume de biogaz dans le sas de stockage 43 même si un tel volume ne représenterait pas un danger pour la sécurité du système 1 et des opérateurs.

Lorsque la première vanne 41 est commandée par le module de contrôle 60, la première vanne 41 peut envoyer un message de confirmation de fermeture au module de contrôle 60 une fois ladite première vanne 41 fermée afin de permettre au module de contrôle 60 de poursuivre la suite de la purge.

Ensuite, dans une étape F6, la deuxième vanne 42 est ouverte afin de vider le sas de stockage 43 et terminer ainsi l'opération de purge en revenant à l'état initial (figure 11). La deuxième vanne 42 peut ensuite être refermée en vue de la purge suivante.

Dans les deux modes, la fraction de partie liquide 100 s'écoulant du sas de stockage 43 via la deuxième vanne 42 est évacué vers l'extérieur du système 1, par exemple vers un réseau d'évacuation des eaux usées.

On notera qu'à tout moment, avantageusement au moins l'une de la première vanne 41 et de la deuxième vanne 42 est fermée afin d'éviter tout fuite de biogaz du système 1 dans l'atmosphère et améliorer ainsi la sécurité du système 1.

On notera que le fonctionnement du système 1 selon le premier mode ou le deuxième mode, même si chaque mode a été décrit selon une forme de réalisation différente du conduit d'évacuation 30, ne dépend pas de la forme ou des dimensions dudit conduit d'évacuation 30. Notamment, le premier mode de réalisation aurait tout aussi bien pu être décrit en référence à la deuxième forme de réalisation (conduit d'évacuation droit) et le deuxième mode de réalisation aurait tout aussi bien pu être décrit en référence à la première forme de réalisation (conduit d'évacuation coudé).

Le sas de stockage 43 formé entre la première vanne 41 et la deuxième vanne 42 permet donc d'évacuer en deux temps tout ou partie de la partie liquide 100 sans risque en isolant du conduit d'acheminement 20 une portion du conduit d'évacuation 30. La commande à distance de la première vanne 41 et de la deuxième vanne 42 par le module de contrôle 60, de préférence de manière automatisée, permet d'éviter à un opérateur de se déplacer pour manipuler manuellement la première vanne 41 et la deuxième vanne 42, augmentant ainsi la sécurité du système 1. En outre, la commande à distance de la première vanne 41 et de la deuxième vanne 42 par le module de contrôle 60, de préférence de manière automatique, permet de simplifier les opérations de purge et donc la maintenance du système 1.

Il est à noter enfin que la présente invention n'est pas limitée aux exemples décrits ci-dessus et est susceptible de nombreuses variantes accessibles à l'homme de l'art. Notamment, la forme et les dimensions du méthaniseur 10, notamment de la cuve 110 et du module de couverture 120, du conduit d'acheminement 20, du conduit d'évacuation 30 et du détecteur 50 tels que représentés sur les figures de façon à illustrer un exemple de réalisation de l'invention ainsi que les matériaux utilisés ne sauraient être interprétés comme limitatifs.

## Revendications

1. Système (1) de méthanisation de matières organiques, ledit système (1) comprenant un méthaniseur (10), un conduit d'acheminement (20), un conduit d'évacuation (30), une première vanne (41) montée dans ledit conduit d'évacuation (30) et un détecteur (50), ledit conduit d'acheminement (20) étant relié fluidiquement au méthaniseur (10) afin d'évacuer le biogaz produit par ledit méthaniseur (10), ledit conduit d'évacuation (30) étant relié fluidiquement au conduit d'acheminement (20) afin de collecter la partie liquide (100) circulant dans le conduit d'acheminement (20) et de la stocker, ledit détecteur (50) comprenant un flotteur (51), placé à l'intérieur du conduit d'évacuation (30) et qui est apte à flotter à la surface de la partie liquide (100) collectée dans ledit conduit d'évacuation (30), et un élément sensible (52), monté en amont de la première vanne (41) et qui est apte à détecter le flotteur (51) lorsque ledit flotteur (51) flotte au droit dudit élément sensible (52) afin de permettre l'évacuation d'au moins une fraction de la partie liquide (100) stockée dans le conduit d'évacuation (30), ledit système (1) étant **caractérisé en ce qu'**il comprend une deuxième vanne (42) montée en aval de la première vanne (41) de sorte à former un sas de stockage (43) de la fraction de partie liquide (100) entre la première vanne (41) et la deuxième vanne (42), ledit sas de stockage (43) étant fluidiquement indépendant du conduit d'acheminement (30) lorsque la première vanne (41) est fermée.

2. Système (1) de méthanisation selon la revendication 1, dans lequel la première vanne (41) et la deuxième vanne (42) sont configurées pour être commandées à distance.

3. Système (1) de méthanisation selon l'une quelconque des revendications précédentes, dans lequel la première vanne (41) et la deuxième vanne (42) sont des électrovannes.

4. Système (1) de méthanisation selon l'une quelconque des revendications précédentes, comprenant un module de contrôle (60) apte à recevoir un signal de détection du détecteur (50) lorsque l'élément sensible (52) détecte le flotteur (51) et à commander l'ouverture et la fermeture de la première vanne (51) et de la deuxième vanne (52).

5. Système (1) de méthanisation selon la revendication précédente, dans lequel le module de contrôle (60) est configuré pour, à réception du signal de détection, fermer la première vanne (41), ouvrir la deuxième vanne (42) lorsque la première vanne (41) a été fermée afin d'évacuer l'au moins une fraction de partie liquide (100) stockée dans le sas de stockage (43), fermer la deuxième vanne (42) lorsque l'au moins une fraction de partie liquide (100) a été évacuée du sas de stockage (43) et ouvrir la première vanne (41).

6. Système (1) de méthanisation selon la revendication 4, dans lequel le module de contrôle (60) est configuré pour, à réception du signal de détection, ouvrir la première vanne (41) pour remplir le sas de stockage (43) avec au moins une fraction de la partie liquide (100), la deuxième vanne (42) étant fermée, fermer la première vanne (41), ouvrir la deuxième vanne (42) lorsque la première vanne (41) a été fermée afin d'évacuer l'au moins une fraction de partie liquide (100) stockée dans le sas de stockage (43) et fermer la deuxième vanne (42) lorsque l'au moins une fraction de partie liquide (100) a été évacuée du sas de stockage (43).

7. Système (1) de méthanisation selon l'une quelconque des revendications précédentes, comprenant un filtre (70) à charbon actif relié au conduit d'acheminement (20) de biogaz.

8. Système (1) de méthanisation selon l'une quelconque des revendications précédentes, dans lequel le conduit d'acheminement (20) comprend un point bas et le conduit d'évacuation (30) est connecté fluidiquement audit conduit d'acheminement (20) au niveau dudit point bas.

9. Procédé d'évacuation de la partie liquide circulant dans le conduit d'acheminement (20) d'un système (1) de méthanisation selon l'une quelconque des revendications précédentes, ledit procédé comprenant, la première vanne (41) étant ouverte et la deuxième vanne (42) étant fermée, les étapes de :
- collecte (E1) de la partie liquide dans le sas de stockage (43),
- détection (E2) du flotteur (51) par l'élément sensible (52) du détecteur (50),
- fermeture (E3) de la première vanne (41),
- ouverture (E4) de la deuxième vanne (42) afin d'évacuer la partie liquide stockée dans le sas de stockage (43),
- fermeture (E5) de la deuxième vanne (42),
- ouverture (E6) de la première vanne (41) afin de permettre de nouveau le stockage de la partie liquide dans le sas de stockage (43).

10. Procédé d'évacuation de la partie liquide circulant dans le conduit d'acheminement (20) d'un système (1) de méthanisation selon l'une quelconque des revendications précédentes, ledit procédé comprenant, la première vanne (41) et la deuxième vanne (42) étant fermées, les étapes de :
- collecte (F1) de la partie liquide dans le conduit d'évacuation (20) en amont de la première vanne (41),
- détection (F2) du flotteur (51) par l'élément sensible (52) du détecteur (50),
- ouverture (F3) de la première vanne (41),
- stockage (F4) de la partie liquide dans le sas de stockage (43),
- fermeture (F5) de la première vanne (41),
- une fois la première vanne (41) fermée, ouverture (F6) de la deuxième vanne (42) afin d'évacuer la partie liquide stockée dans le sas de stockage (43).

## Patentansprüche

1. System (1) zur Methanisierung organischer Materialien, wobei das System (1) einen Methanisierer (10), eine Zufuhrleitung (20), eine Ableitungsleitung (30), ein erstes Ventil (41), das in der Ableitungsleitung (30) angebracht ist und einen Detektor (50) umfasst, wobei die Zufuhrleitung (20) mit dem Methanisierer (10) fluidisch verbunden ist, um das vom Methanisierer (10) produzierte Biogas abzuleiten, wobei die Ableitungsleitung (30) mit der Zufuhrleitung (20) fluidisch verbunden ist, um den Flüssiganteil (100) zu sammeln, der in der Zufuhrleitung (20) zirkuliert und ihn zu speichern, wobei der Detektor (50) einen Schwimmer (51) umfasst, der im Inneren der Ableitungsleitung (30) platziert ist und der imstande ist, an der Oberfläche des Flüssiganteils (100) zu schwimmen, der in der Ableitungsleitung (30) gesammelt ist, und ein sensibles Element (52), das stromaufwärts zum ersten Ventil (41) angebracht ist und das imstande ist, den Schwimmer (51) zu ermitteln, wenn der Schwimmer (51) neben dem sensiblen Element (52) schwimmt, um die Ableitung mindestens eines Teils des in der Ableitungsleitung (30) gespeicherten Flüssiganteils (100) zu gestatten, wobei das System (1) **dadurch gekennzeichnet ist, dass** es ein zweites Ventil (42) umfasst, das stromabwärts zum ersten Ventil (41) derart angebracht ist, dass eine Speicherschleuse (43) des Teils des Flüssiganteils (100) zwischen dem ersten Ventil (41) und dem zweiten Ventil (42) gebildet wird, wobei die Speicherschleuse (43) von der Zufuhrleitung (30) fluidisch unabhängig ist, wenn das erste Ventil (41) geschlossen ist.

2. Methanisierungssystem (1) nach Anspruch 1, wobei das erste Ventil (41) und das zweite Ventil (42) ausgelegt sind, um ferngesteuert zu sein.

3. Methanisierungssystem (1) nach einem der vorangehenden Ansprüche, wobei das erste Ventil (41) und das zweite Ventil (42) Magnetventile sind.

4. Methanisierungssystem (1) nach einem der vorangehenden Ansprüche, das ein Steuermodul (60) umfasst, das imstande ist, vom Detektor (50) ein Detektionssignal zu empfangen, wenn das sensible Element (52) den Schwimmer (51) ermittelt und das Öffnen und das Schließen des ersten Ventils (51) und des zweiten Ventils (52) zu befehlen.

5. Methanisierungssystem (1) nach vorangehendem Anspruch, wobei das Steuermodul (60) ausgelegt ist, um beim Empfang des Detektionssignals das erste Ventil (41) zu schließen, das zweite Ventil (42) zu öffnen, wenn das erste Ventil (41) geschlossen wurde, um den mindestens einen Teil des Flüssiganteils (100) abzuleiten, der in der Speicherschleuse (43) gespeichert ist, das zweite Ventil (42) zu schließen, wenn mindestens ein Teil des Flüssiganteils (100) aus der Speicherschleuse (43) abgeleitet wurde und das erste Ventil (41) zu öffnen.

6. Methanisierungssystem (1) nach Anspruch 4, wobei das Steuermodul (60) ausgelegt ist, um beim Empfang des Detektionssignals das erste Ventil (41) zu öffnen, um die Speicherschleuse (43) mit mindestens einem Teil des Flüssiganteils (100) zu füllen, wobei das zweite Ventil (42) geschlossen ist, das erste Ventil (41) zu schließen, das zweite Ventil (42) zu öffnen, wenn das erste Ventil (41) geschlossen wurde, um den mindestens einen Teil des Flüssiganteils (100) abzuleiten, der in der Speicherschleuse (43) gespeichert ist, und das zweite Ventil (42) zu schließen, wenn der mindestens eine Teil des Flüssiganteils (100) aus der Speicherschleuse (43) abgeleitet wurde.

7. Methanisierungssystem (1) nach einem der vorangehenden Ansprüche, das einen Aktivkohlefilter (70) umfasst, der mit der Biogas-Zufuhrleitung (20) verbunden ist.

8. Methanisierungssystem (1) nach einem der vorangehenden Ansprüche, wobei die Zufuhrleitung (20) einen niedrigen Punkt umfasst und die Ableitungsleitung (30) an die Zufuhrleitung (20) im Bereich des niedrigen Punkts fluidisch angeschlossen ist.

9. Verfahren zur Ableitung des Flüssiganteils, der in der Zufuhrleitung (20) eines Methanisierungssystems (1) nach einem der vorangehenden Ansprüche zirkuliert, wobei das Verfahren, wobei das erste Ventil (41) geöffnet und das zweite Ventil (42) geschlossen ist, die folgenden Schritte umfasst:
- Sammeln (E1) des Flüssiganteils in der Speicherschleuse (43),
- Ermitteln (E2) des Schwimmers (51) durch das sensible Element (52) des Detektors (50),
- Schließen (E3) des ersten Ventils (41),
- Öffnen (E4) des zweiten Ventils (42), um den in der Speicherschleuse (43) gespeicherten Flüssiganteil abzuleiten,
- Schließen (E5) des zweiten Ventils (42),
- Öffnen (E6) des ersten Ventils (41), um erneut das Speichern des Flüssiganteils in der Speicherschleuse (43) zu gestatten.

10. Verfahren zur Ableitung des Flüssiganteils, der in der Zufuhrleitung (20) eines Methanisierungssystems (1) nach einem der vorangehenden Ansprüche zirkuliert, wobei das Verfahren, wobei das erste Ventil (41) und das zweite Ventil (42) geschlossen sind, die folgenden Schritte umfasst:
- Sammeln (F1) des Flüssiganteils in der Ableitungsleitung (20) stromaufwärts zum ersten Ventil (41),
- Ermitteln (F2) des Schwimmers (51) durch das sensible Element (52) des Detektors (50),
- Öffnen (F3) des ersten Ventils (41),
- Speichern (F4) des Flüssiganteils in der Speicherschleuse (43),
- Schließen (F5) des ersten Ventils (41),
- sobald das erste Ventil (41) geschlossen ist, Öffnen (F6) des zweiten Ventils (42), um den in der Speicherschleuse (43) gespeicherten Flüssiganteil abzuleiten.

## Claims

1. An organic matter methanisation system (1), said system (1) comprising a methaniser (10), a conveying duct (20), a discharge duct (30), a first valve (41) mounted in said discharge duct (30) and a detector (50), said conveying duct (20) being fluidly connected to the methaniser (10) in order to discharge the biogas produced by said methaniser (10), said discharge duct (30) being fluidly connected to the conveying duct (20) in order to collect the liquid part (100) circulating in the conveying duct (20) and to store it, said detector (50) comprising a float (51), placed inside the discharge duct (30) and which is capable of floating on the surface of the liquid part (100) collected in said discharge duct, and a sensitive element (52), mounted upstream of the first valve (41) and which is capable of detecting the float (51) when said float (51) floats flush with said sensitive element (52) in order to allow discharge of at least one fraction of the liquid part (100) stored in the discharge duct (30), said system (1) being **characterised in that** it comprises a second valve (42) mounted downstream of the first valve (41) so as to form a storage lock (43) of the fraction of liquid part (100) between the first valve (41) and the second valve (42), said storage lock (43) being fluidly independent of the conveying duct (30) when the first valve (41) is closed.

2. The methanisation system (1) according to claim 1, wherein the first valve (41) and the second valve (42) are configured to be remotely controlled.

3. The methanisation system (1) according to any of the preceding claims, wherein the first valve (41) and the second valve (42) are solenoid valves.

4. The methanisation system (1) according to any of the preceding claims, comprising a control module (60) capable of receiving a detection signal from the detector (50) when the sensitive element (52) detects the float (51) and controlling the opening and closing of the first valve (51) and the second valve (52).

5. The methanisation system (1) according to the preceding claim, wherein the control module (60) is configured to, upon receiving the detection signal, close the first valve (41), open the second valve (42) when the first valve (41) has been closed in order to discharge the at least one fraction of liquid part (100) stored in the storage lock (43), close the second valve (42) when the at least one fraction of liquid part (100) has been discharged from the storage lock (43) and open the first valve (41).

6. The methanisation system (1) according to claim 4, wherein the control module (60) is configured to, upon receiving the detection signal, open the first valve (41) to fill the storage lock (43) with at least one fraction of the liquid part (100), the second valve (42) being closed, close the first valve (41), open the second valve (42) when the first valve (41) has been closed to discharge the at least one fraction of liquid part (100) stored in the storage lock (43) and close the second valve (42) when the at least one fraction of liquid part (100) has been discharged from the storage lock (43).

7. The methanisation system (1) according to any of the preceding claims, comprising an activated carbon filter (70) connected to the biogas conveying duct (20).

8. The methanisation system (1) according to any of the preceding claims, wherein the conveying duct (20) comprises a low point and the discharge duct (30) is fluidly connected to said conveying duct (20) at said low point.

9. A method for discharging the liquid part circulating in the conveying duct (20) of a methanisation system (1) according to any of the preceding claims, said method comprising, with the first valve (41) open and the second valve (42) closed, the steps of:
- collecting (E1) the liquid part in the storage lock (43),
- detecting (E2) the float (51) by the sensitive element (52) of the detector (50),
- closing (E3) the first valve (41),
- opening (E4) the second valve (42) in order to discharge the liquid part stored in the storage lock (43),
- closing (E5) the second valve (42),
- opening (E6) the first valve (41) to allow storage of the liquid part in the storage lock (43) again.

10. The method for discharging the liquid part circulating in the conveying duct (20) of a methanisation system (1) according to any of the preceding claims, said method comprising, with the first valve (41) and the second valve (42) closed, the steps of:
- collecting (F1) the liquid part in the discharge duct (20) upstream of the first valve (41),
- detecting (F2) the float (51) by the sensitive element (52) of the detector (50),
- opening (F3) the first valve (41),
- storing (F4) the liquid part in the storage lock (43),
- closing (F5) the first valve (41),
- once the first valve (41) closed, opening (F6) the second valve (42) in order to discharge the liquid part stored in the storage lock (43).
